(19) Europäisches Patentamt
European Patent Office
Office européen des brevets

(11) Veröffentlichungsnummer: **0 065 200**
**B1**

(12) # EUROPÄISCHE PATENTSCHRIFT

(45) Veröffentlichungstag der Patentschrift: **25.02.87**

(51) Int. Cl.⁴: **C 07 D 207/24**

(21) Anmeldenummer: **82103847.8**

(22) Anmeldetag: **05.05.82**

(54) **Delta-1-Pyrrolin-thiolactimäther und Verfahren zu ihrer Herstellung.**

(30) Priorität: **07.05.81 DE 3117979**

(43) Veröffentlichungstag der Anmeldung: **24.11.82 Patentblatt 82/47**

(45) Bekanntmachung des Hinweises auf die Patenterteilung: **25.02.87 Patentblatt 87/9**

(84) Benannte Vertragsstaaten: **AT BE CH DE FR GB IT LI LU NL SE**

(56) Entgegenhaltungen: **DE-A-2 100 422**

**Tetrahedron letters 23, 2947-2950 (1982)**

(73) Patentinhaber: **HOECHST AKTIENGESELLSCHAFT, Postfach 80 03 20, D-6230 Frankfurt am Main 80 (DE)**

(72) Erfinder: **Beck, Gerhard, Dr., Gustav- Freytag-Strasse 24, D-6000 Frankfurt am Main (DE)**
Erfinder: **Bartmann, Wilhelm, Dr., Am Dachsbau 5, D-6232 Bad Soden am Taunus (DE)**
Erfinder: **Knolle, Jochen, Dr., Höchster Strasse 21, D-6239 Kriftel (DE)**
Erfinder: **Rupp, Richard Helmut, Dr., Röderweg 16a, D-6240 Königstein/Taunus (DE)**

**EP 0 065 200 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Prostacyclin $PGI_2$, ein 1976 isolierter Naturstoff aus der Familie der Prostaglandine, zeichnet sich durch seine stark ausgeprägten thrombocytenaggregationshemmenden Eigenschaften aus (The Lancet 1977, 18). Außerdem vermag $PGI_2$ einige Blutgefäße, z.B. Coronararterien, zu relaxieren (Prostaglandins 13, 3, 1977), so daß es zur Therapie und Prophylaxe von Thrombosen und Infarkten Verwendung finden kann. $PGI_2$ zeigt weiter eine ausgeprägte blutdrucksenkende Wirkung (z.B. IRCS Med. Sci. 6, 392 (1978)).

Gegenstand der vorliegenden Erfindung sind neue Verbindungen der allgemeinen Formel I

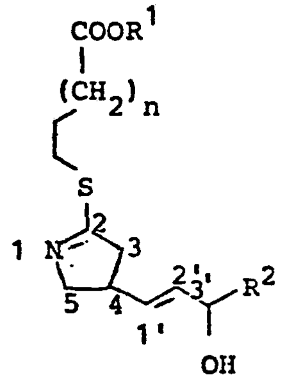

I

die eine spezifischere Wirkung und/oder längere Wirkungsdauer als $PGI_2$ besitzen und in welcher bedeuten:

$R^1$ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen oder ein physiologisch verträgliches Metall-, $NH_4$- oder ein Ammoniumion, das sich von einem primären, sekundären oder tertiären Amin ableitet, oder ein Tetraalkylammoniumion

$R^2$ einen Phenylrest, der 1-3-fach substituiert sein kann mit Halogen, Trifluormethyl, Alkyl und/oder Alkoxy mit je 1-6 C-Atomen, oder einen unsubstituierten cycloaliphatischen Kohlenwasserstoffrest mit 3-8 Kohlenstoffatomen oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen, oder einem Phenylmethyloxyrest,

b) Halogen, unsubstituiertem Cycloalkyl mit 3-7 C-Atomen, Phenyl oder einem α- oder-β-Thienyl- oder α - oder β-Furylrest, die ihrerseits im Kern 1-3 fach substituiert sein können mit Halogen, Trifluormethyl, Alkyl und/oder Alkoxy mit 1-6 C-Atomen,

c) einem unsubstituierten Cycloalkoxyrest mit 3-7 Kohlenstoffatomen, einem Phenoxy- oder einem α- oder β-Thienyloxyrest, wobei die genannten aromatischen Reste ihrerseits im Kern 1-3-fach substituiert sein können mit Halogen, Trifluormethyl, Alkyl und/oder Alkoxy mit je 1 - 6 C-Atomen, und

n die Zahl 0, 1, 2, 3 oder 4.

Unter den Substituenten $R^1$ sind bevorzugt:

Wasserstoff, geradkettiges oder verzweigtes Alkyl mit bis zu 8 C-Atomen, ein geradkettiger oder verzweigter ungesättigter, aliphatischer Kohlenwasserstoffrest mit bis zu 4 C-Atomen, ein cycloaliphatischer Kohlenwasserstoffrest mit 5-7 C-Atomen, ein araliphatischer Kohlenwasserstoffrest mit 8 oder 9 C-Atomen oder ein Ammoniumion, das sich von einem primären, sekundären oder tertiären Amin ableitet, insbesondere:

Wasserstoff, Methyl, Äthyl, n-Butyl, n-Pentyl, n-Hexyl, n-Heptyl, 2-Propyl, 2-Butyl, 2-Pentyl, 3-Hexyl, 2-Methyl-propyl, 2-Methylbutyl, 4,4-Dimethylpentyl, 5,5-Dimethyl-hexyl, Cyclopentyl, Cyclohexyl, Cycloheptyl, Methylammonium, Dicyclohexylammonium, Tris-(hydroxymethyl)-methylammonium).

Unter den Substituenten $R^2$ sind die im folgenden ausgeführten besonders bevorzugt:

unsubstituiertes Phenyl oder mit Halogen, Trifluormethyl, $C_{1-4}$-Alkyl oder $C_{1-4}$-Alkoxy einfach substituiertes Phenyl, geradkettiges oder verzweigtes $C_{3-7}$-Alkyl, das substituiert sein kann mit $C_5$-$C_7$-Cycloalkyl, mit $C_{1-3}$-Alkoxy, mit Phenoxy oder Halogenphenoxy, mit Thienyloxy oder Halogenthienyloxy, mit Cyclohexyloxy, mit Thienyl, mit Halogenthienyl oder mit Furyl, insbesondere die Reste:

n-Pentyl, 1,1-Dimethylpentyl, Cyclopentylmethyl, Cyclohexylmethyl, 1,1-Dimethyl-2-äthoxy-äthyl, 1,1-Dimethyl-2-methoxy-äthyl, 1,1-Dimethyl-cyclohexyloxymethyl, 1-Fluorpentyl, 1-Chlorpentyl, 5-Fluorpentyl, 5-Chlorpentyl, 3-Thienyl-2-äthyl, 2-Thienyl-2-äthyl, 3-(2-Chlorthienyl)-2-äthyl, 2-(5-Chlorthienyl)-2-äthyl, Phenoxymethyl, 3-Chlorphenoxy-methyl, 2-Thienyloxymethyl, 3-(2-Chlorthienyl)-oxy-methyl, 2-(5-Chlorthienyl)-oxymethyl, 3-Furyl-2-äthyl, 2-Furyl-2-äthyl, 2,2,3,3,-Tetrafluorcyclobutyl-2-äthyl, Phenyl, 3-Chlorphenyl, 3-Trifluormethylphenyl.

n hat bevorzugt die Bedeutung 0, 1 oder 2.

Gegenstand der Erfindung ist auch ein Verfahren zur Herstellung der Verbindungen der Formel I, das dadurch gekennzeichnet ist, daß man

a) das Lactam der Formel II

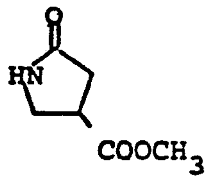

II

nach üblichen Methoden umwandelt in das Thiolactam der Formel III,

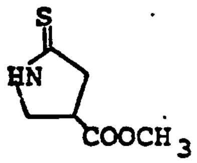

III

b) das Thiolactam der Formel III durch Reduktion der Esterfunktion in den Thiolactamalkohol IV überführt,

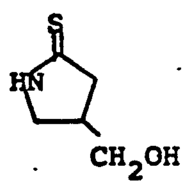

IV

c) den Thiolactamalkohol der Formel IV mit einer Verbindung der Formel V,

$$Hal-CH_2-CH_2-(CH_2)_n-COOR^1 \qquad V$$

worin $R^1$ und n die zur Formel I genannte Bedeutung haben und Hal Jod, Chlor oder Brom bedeutet, alkyliert zu einer Verbindung der Formel VI

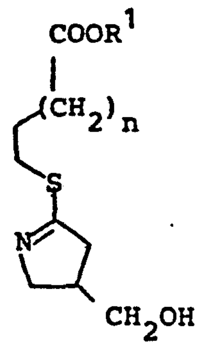

VI

d) die Verbindung der Formel VI oxydiert zu einem Aldehyd der Formel VII

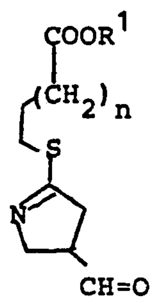

VII

worin $R^1$ und n die oben genannte Bedeutung haben,

e) den Aldehyd der Formel VII umsetzt mit einem Phosphonat der Formel VIII

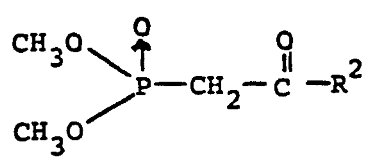

VIII

worin $R^2$ die zur Formel I gegebene Bedeutung hat, zu einem Enon der Formel IX

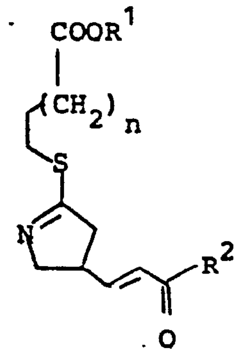

IX

worin $R^1$, $R^2$ und n die zur Formel I gegebene Bedeutung haben,

f) das Enon der Formel IX in bekannter Weise mit einem Redaktionsmittel zu einem Epimerengemisch der Alkohole der Formel I reduziert, worin $R^1$, $R^2$ und n die zur Formel I gegebene Bedeutung haben,

g) gegebenenfalls eine Verbindung der Formel I, worin $R^1$ nicht gleich Wasserstoff oder ein Kation ist, verseift zu einer Verbindung der Formel I, worin $R^1$ Wasserstoff oder ein physiologisch verträgliches Kation wie oben zur Formel I definiert, bedeutet,

h) gegebenenfalls in einer Verbindung der Formel I, worin $R^1$ gleich Wasserstoff oder ein physiologisch verträgliches Metall-, $NH_4$- oder Ammoniumion bedeutet, das sich von einem primären, sekundären oder tertiären Amin ableitet, und $R^2$ und n die zur Formel I gegebenen Bedeutungen haben, das Kation $R^1$ gegen ein anderes unter die obige Definition von $R^1$ fallendes Kation austauscht.

Für die Herstellung des im erfindungsgemäßen Verfahren als Ausgangsmaterial verwendeten Lactams II kann man analog dem Verfahren arbeiten, das in P.L. Paytash, E. Sparrow, I.C. Gathe, J. Am. Chem. Soc. 72, 1415 (1950) beschrieben ist.

Das Thiolactam der Formel III läßt sich aus dem Lactam der Formel II durch Umsetzung mit schwefelübertragende Reagentien, wie z.B. Phosphorpentasulfid, Phosphorpentasulfid/ Calciumoxid, Phosphorpentasulfid-Pyridin-Komplex oder Phosphorpentasulfid-Anisol-Komplex in inerten Lösungsmitteln, wie z.B. Toluol, Dimethoxyäthan oder Pyridin nach literaturbekannten Methoden herstellen (siehe z.B. Bull. Soc. Chim. Belg. 87, (3), 229 (1978)). Den Thiolactam-Alkohol der Formel IV erhält man, wenn man das Thiolactam der Formel III mit einem komplexen Metallhydrid, vorzugsweise einem Alkaliboranat wie Natrium-, Kalium- oder Lithium-borhydrid umsetzt. Zur Darstellung von Thiolactimetherndet Formel VI wird das Thiolactam der Formel IV mit Alkylhalogeniden der Formel V alkyliert. Diese Reaktion wird in einem inerten Lösungsmittel, wie z.B. Toluol, Tetrahydrofuran, Dimethoxyethan oder Dimethylformamid in Gegenwart einer Base, wie z.B. Pyridin, Triethylamin, Kaliumcarbonat oder Natriumhydrid bei 15 - 180°C durchgeführt. Eine bevorzugte Ausführungsform dieser Reaktion besteht darin, daß man das S-Kaliumsalz des Thiolactams der Formel IV mit Kaliumcarbonat in Dimethylformamid herstellt und dazu eine Lösung der Verbindung der Formel V in Dimethylformamid tropft und 1 - 6 Stunden bei 90 - 160°C rührt. Die Oxidation eines Alkohols der Formel VI zu einem Aldehyd der Formel VII kann durch Oxidationsmittel wie Pyridiniumchlorochromat in inerten Lösungsmitteln wie Methylenchlorid oder Chloroform erfolgen. Eine weitere Möglichkeit der Oxidation besteht in der Reaktion mit Thioanisol/$Cl_2$/Trimethylamin in Tetrachlorkohlenstoff. Der Aldehyd der Formel VII wird zweckmäßigerweise ohne weitere Reinigung weiterverarbeitet.

Im weiteren wird ein Aldehyd der Formel VII nach Horner-Emmons-Wittig mit einem Phosphonsäureester der Formel VIII zu einem ungesättigten Keton der Formel IX umgesetzt, wobei eine bevorzugte Ausführungsform darin besteht, daß man das Natriumsalz des Phosphonsäureesters der Formel VIII mit Natriumhydrid in Dimethoxyethan herstellt und anschliessend den Aldehyd der Formel VII zugibt und bei Raumtemperatur 2 - 6 Stunden reagieren läßt. Die Phosphonsäureester der Formel VIII können nach literaturbekannten Verfahren (siehe z.B. J. Am. Chem. Soc. 88, 5654 (1966) hergestellt werden.

Die Verbindungen der Formel I erhält man in Form ihrer Epimerengemische, wenn man ein Enon der Formel IX mit einem komplexen Metallhydrid, vorzugsweise mit Alkaliboranat oder mit D,L-Isobornyloxyaluminium-isopropoxid reduziert.

Verbindungen der Formel I worin $R^1$ nicht Wasserstoff oder ein Kation darstellt, können in üblicher Weise in alkalischem Medium zu Verbindungen der Formel 1, worin $R^1$ Wasserstoff oder ein Kation bedeutet, verseift werden, z.B. mit NaOH oder KOH in einem niedermolekularen Alkohol wie Methanol oder Äther wie Dimethoxyäthan oder THF, gegebenenfalls in Gegenwart von Wasser. Vorteilhafterweise benutzt man die äquimolare Menge oder einen sehr geringen Überschuß an Alkalihydroxid, so daß man das Alkalisalz der Formel I ($R^1$ = Alkalimetallion) durch Verdampfen des Lösungsmittels erhält, vorzugsweise durch Gefriertrocknung.

Das Alkalikation läßt sich an Ionenaustauschern in üblicher Weise gegen beliebige Kationen austauschen.

Dazu läßt man die Lösung des Alkalisalzes eines erfindungsgemäßen Imino-Hetero-Prostacyclinderivates durch eine mit einem Kationenaustauscher, wie z.B. Amberlite CG-50 oder Dowex CCR-2 gefüllte Säule laufen. Der Kationenaustauscher ist mit dem erwünschten Kation beladen, z.B. mit einem Ammoniumion, das sich von einem primären, sekundären oder tertiären Amin ableitet. Das erwünschte Salz erhält man durch Eindampfen des Eluats.

Man kann Verbindungen der Formel I, bei denen $R^1 = NH_4$ oder ein Ammoniumion bedeutet, das sich von einem primären, sekundären oder tertiären Amin ableitet, auch herstellen, indem man Verbindungen der Formel I, bei denen $R^1$ = Wasserstoff bedeutet, in einer alkoholischen Lösung mit einer äquimolaren Menge des entsprechenden Amins versetzt und das Lösungsmittel eindampft.

Verbindungen der Formel I, worin $R^1$ Wasserstoff oder ein Kation bedeutet, lassen sich zu Verbindungen der Formel I verestern, worin $R^1$ die übrigen zur Formel I gegebenenen Bedeutungen hat. So kann man z.B. Verbindungen der Formel I mit $R^1$ = H bei Temperaturen zwischen -40° und +20°C mit einem Diazoalkan der Formel $R^1 = N_2$ mit $R^1$ = Alkyl verestern, wobei die üblichen Lösungsmittel wie z.B, Diäthylether, Tetrahydrofuran, Chloroform oder nieder-molekulare Alkohole wie Methanol verwendet werden können. Die resultierenden Ester können in einfacher Weise durch Eindampfen des Lösungsmittels isoliert und gegebenenfalls chromatographisch gereinigt werden. Eine Veresterungsmethode besteht darin, daß man Salze der Verbindungen der Formel I ($R^1$ = Kation) in Gegenwart einer Base wie z.B. eines Metallalkoholates oder Metallcarbonates in einem geeigneten Lösungsmittel mit einem Alkylierungsmittel $R^1$-Z umsetzt. Als Metallalkoholate kommen z.B. Natriummethylat, Natriumäthylat oder Kaliumtertiärbutylat in Betracht, als Carbonat eignet sich z.B. Calciumcarbonat. Als geeignetes Lösungsmittel kommen Alkohole wie z.B. Methanol oder tert. Butanol, Äther wie Tetrahydrofuran oder 1,2-Dimethoxyäthan und insbesondere dipolare aprotische Lösungsmittel wie Dimethylformamid, Dimethylsulfoxyd, Acetonitril, oder N-Methylpyrrolidon in Betracht. In der Formel $R^1$ - Z bedeutet Z vorzugsweise Brom oder Jod oder einen Sulfonsäurerest. Zur Darstellung von Estern der Formel I ($R^1$ = Alkyl) eignet sich auch die Methode der Umesterung mit Überschuß an Alkoholen wie z.B. Methanol, Äthanol, Isopropanol.

Die Verbindungen der Formel I fallen als Racemat bezüglich der Stellung am Kohlenstoffatom 4 des Pyrrolinrings sowie als α /β-Isomere bezüglich des Kohlenstoffatoms 3' an. Die Trennung der α /β-Isomeren erfolgt auf der Stufe der Endprodukte der Formel I. Die Trennung des Racemats bezüglich des Kohlenstoffatoms 4 des Δ1-Pyrrolinrings kann nach jeder Reaktionsstufe erfolgen, oder es wird das optisch aktive (+) oder (-) Lactam II eingesetzt. Das bedeutet, daß alle beschriebenen Reaktionen mit Epimerengemischen, reinen Epimeren oder optisch aktiven Antipoden durchgeführt werden können. Die beanspruchten Verbindungen der Formel I umfassen daher Diastereomerengemische, reine Diastereomere, Epimerengemische und reine Epimeren.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Außer den in den Beispielen beschriebenen Verbindungen lassen sich nach den erfindungsgemäßen Verfahren die folgenden Verbindungen herstellen:

2(1-Thia-5-carboxy-pentyl)-4(3-hydroxy-1-octenyl)-Δ1-pyrrolin
2(1-Thia-4-carboxy-butyl)-4(3-hydroxy-1-octenyl)- Δ1-pyrrolin
2(1-Thia-6-carboxy-hexyl)-4(3-hydroxy-1-octenyl)- Δ1-pyrrolin
2(1-Thia-4-methoxycarbonyl-butyl)-4(3-hydroxy-1-octenyl)-Δ1-pyrrolin
2(1-Thia-5-methoxycarbonyl-pentyl)-4(3-hydroxy-1-octenyl)-Δ1-pyrrolin
2(1-Thia-4-methoxycarbonyl-butyl)-4(3-hydroxy-5(2-furyl)-1-pentenyl)- Δ1-pyrrolin
2(1-Thia-4-methoxycarbonyl-butyl)-4(3-hydroxy-4,4-di-methyl-4-cyclohexyloxy-1-butenyl)- Δ1-pyrrolin
2(1-Thia-4-methoxycarbonyl-butyl)-(3-hydroxy-3-phenyl-1-propenyl)- Δ1-pyrrolin
2(1-Thia-4-äthoxycarbonyl-butyl)-4(3-hydroxy-4(3-tri-fluormethyl-phenyloxy)l-butenyl)- Δ1-pyrrolin
2(1-Thia-4-cyclohexyloxycarbonyl-butyl)-4(3-hydroxy-1-octenyl)- Δ1-pyrrolin
2(1-Thia-7-methoxycarbonyl-heptyl)-4(3-hydroxy-1-octenyl-Δ1-pyrrolin
2(1-Thia-4-äthoxycarbonyl-butyl)-4(3-hydroxy-1-nonenyl)-Δ1-pyrrolin
2(1-Thia-4-methoxycarbonyl-butyl-4(3-hydroxy-1-decenyl)-Δ1-pyrrolin

Die Verbindungen der Formel I zeichnen sich aus durch hemmende Wirkung auf die Thrombocytenaggregation, Relaxation der Gefäßwand, sowie blutdrucksenkende Eigenschaften. Sie können daher als Arzneimittel angewandt werden. Die Anwendung der Verbindungen der Formel I als Blutdrucksenker erfolgt im Tagesdosisbereich von 0,01 mg/kg - 0,5 mg/kg, vorzugsweise 0,05 mg/kg bis 0,1 mg/kg bei i.v. Applikation bzw. im Tagesdosisbereich von 0,05 mg/kg - 2 mg/kg vorzugsweise 0,1 - 1 mg/kg bei oraler Applikation. Zur Relaxation der Gefäßwand, besonders der Coronararterien sowie zur Hemmung der Thrombocytenaggregation kommen die gleichen Tagesdosen, teilweise auch niedere Dosierungen wie oben angegeben in Betracht.

Die Verbindungen sind bei Säugetieren einschließlich Menschen sowie bestimmten Nutztieren, z.B. Hunden und Schweinen, auch brauchbar zur Verminderung und Steuerung übermäßiger Magensaftsekretion, womit die Bildung von Magen/ Darmgeschwüren vermindert oder vermieden wird und die Heilung solcher bereits vorhandener Geschwüre beschleunigt werden kann. Zu diesem Zweck werden die Verbindungen intravenös, subkutan oder intramuskulär injiziert oder infundiert. Das Dosierungsschema für das Prostaglandin hängt bei dieser Behandlung von verschiedenen Faktoren ab einschließlich Typ, Alter, Gewicht, Geschlecht und

medizinischem Zustand des Patienten, dem Dosierungsschema des entzündungshemmenden Synthetase-Inihitors und der Empfindlichkeit des Patienten auf den Synthetase-Inhibitor bezüglich der Magen/Darmwirkung. So empfindet z.B. nicht jeder Patient, der eine entzündungshemmende Substanz benötigt, die gleichen unangenehmen gastrointestinalen Effekte. Diese ändern sich vielmehr häufig in Art und Ausmaß. Es liegt daher im Erfahrungsbereich des Arztes oder Tierarztes, festzustellen, ob die Verabreichung der entzündungshemmenden Substanz unerwünschte gastrointestinale Effekte beim Mensch oder Tier erzeugt, und die wirksame Menge des Prostaglandins zu verschreiben, mit der diese Effekte im wesentlichen eliminiert werden können. Einzelne Vertreter dieser Substanzen eignen sich zur Behandlung von Asthma. Sie sind beispielsweise nützlich als Bronchiendilatoren oder als Inhibitoren von Mediatoren wie z.B. SRS-A und Histamin, die aus durch einen Antigen/ Antikörper-Komplex aktivierten Zellen freigesetzt werden. Die Verbindungen bekämpfen daher Krämpfe und erleichtern das Atmen bei Krankheitszuständen wie Bronchitis, Pneumonie und Emphysem. Für diese Zwecke werden die Verbindungen in verschiedenen Dosierungsformen verabreicht, z.B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die erfindungsgemäßen Verbindungen der Formel I können als freie Säuren, oder in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther, oder auch Polyäthern wie z.B. Polyäthylenglykol oder auch in Gegenwart anderer pharmakologisch unbedenklicher Polymerträger wie z.B. Polyvinylpyrrolidon zur Anwendung gelangen.

Als Zubereitungen können die üblichen galenischen Infusions-oder Injektionslösungen und Tabletten sowie örtlich anwendbare Zubereitungen wie Cremes, Emulsionen, Suppositorien oder Aerosole in Frage kommen.

Die Verbindungen der Formel VI, VII und IX sind neue wertvolle Zwischenprodukte für die Herstellung von Verbindungen der Formel I.

**Beispiel 1**

**4-Methoxycarbonyl-pyrrolidin-2-thion III**

10 g 4-Methoxycarbonyl-pyrrolidin-2-on II (hergestellt wie in P.L. Paytash, E. Sparrow, I.C. Gathe, J. Am. Chem. Soc. 72, 1415 (1950) beschrieben) werden in 170 ml abs. Toluol in der Wärme gelöst. Dann gibt man nacheinander 16,7 g $P_4S_{10}$ x 4 Pyridin (Riedel de Haen, Hannover, West Germany) und 42 g Kieselgur (als Filterhilfe) zu. Bei 90-100°C erwärmt man anschließend 3 Stunden unter Rühren und Stickstoff als Schutzgas. Dann wird abgesaugt und der Filterrückstand mit heißem Toluol nachgewaschen. Das Filtrat wird im Vakuum eingeengt.

Ausbeute: 7,64 g hellgelbe Kristalle Fp. 106 - 109° C (70 % d. Th.) $C_6H_9NSO_2$ MG = 159

NMR ($CDCl_3$): δ ppm 3,2 (s,3H) $COOCH_3$; 3,1 - 4,1 (m, 5H) HN-$CH_2$, -$CH_2$CS, =CH-$COOCH_3$, 8,3 (breites s, 1H) CSNH

$R_f$-wert: Cyclohexan/Aceton 1:2: 0,80

**Beispiel 2**

**4-Hydroxymethyl-pyrrolidin-2-thion IV**

24,1 g 4-Methoxycarbonyl-pyrrolidin-2-thion III werden in 250 ml Methanol gelöst. In dieser Lösung tropft man unter Rühren bei 0 - 10°C eine Lösung von 12 g Natrium-borhydrid in 20 ml Eiswasser vorsichtig zu. Anschließend rührt man 2 Stunden bei 10°C und 1 Std. bei Raumtemperatur. Dann säuert man mit halb konz. Salzsäure unter Eiskühlung auf pH 1-2 an. Das ausgefallene Natriumchlorid wird über Kieselgur abgesaugt, mit wenig kaltem Methanol gewaschen, das Filtrat i. Vak. eingeengt. Der Rückstand wird mit Essigester + 5 % Methanol aufgenommen, gekühlt und erneut abgesaugt. Das Filtrat wird eingeengt, mit etwas Aceton versetzt und über Nacht i. Kühlschrank aufbewahrt. Die ausgefallenen Kristalle werden bei 0° C abgesaugt.

Ausbeute: 15,4 g (78 % d. Th.) $C_5H_9NSO$ MG = 131 weiße Kristalle Fp. 108 - 111°C

NMR ($CDCl_3$): δ ppm 3,5 - 3,8 (m, 2H) $CH_2OH$, CSNH-$CH_2$; 1,6 (breites s, 1H) OH; 2,6 - 3,0 (m, 3H) -$CH_2$

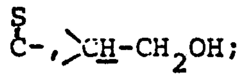

7,7 (breites s, 1H) -CSNH
Rf-wert: Cyclohexan/Aceton = 1:1: 0,28-

**Beispiel 3**

**2(1-Thia-5-äthoxycarbonyl-pentyl)-4-hydroxy-methyl-Δ1 -pyrrolin VI (n = 2)**

Zu 2,7 g Kaliumcarbonat gepulvert in 10 ml abs. Dimethylformamid werden unter Rühren 5,1 g 4-Hydroxymethyl-pyrro-lidin-2-thion IV in 15 ml abs. DMF (Dimethylformid) getropft. Anschließend wird unter Stickstoff 30 Minuten gerührt auf 100°C erhitzt und 8,2 g ω -Bromvaleriansäureäthylester in 6 ml abs. DMF zugetropft. Nach 2 1/2 Std. bei 100°C ist die Reaktion beendet.
Das Lösungsmittel (DMF) wird im Vakuum entfernt und der Rückstand in Essigester und $H_2O$ aufgenommen, mit 2N Salzsäure neutralisiert, die Wasserphase abgetrennt. Die Essigesterphase wird noch mehrmals mit Wasser/gesättigter NaCl-Lösung gewaschen. Die Vereinigten EE-Phasen werden getrocknet und eingeengt i. Vakuum.
Ausbeute: 14,3 g bräunliches Öl. Über eine Merck-Fertigsäule Größe C (Korngröße $SiO_2$ 0,063 - 0,2 mm) chromatographiert. Laufmittel Cyclohexan/Aceton = 2: 1 Fraktion 240 - 370 ergab 5,5 g (55 % d. Th.) VI $C_{12}H_{21}NSO_3$ MG = 259
NMR ($CDCl_3$): δ ppm 1,2 (t, 3H) $CO_2CH_2CN_3$; 1,5 - 2,0 (m, 4H) $-CH_2-CH_2-$; 2,1 - 2,5 (m, 2H) $CH_2-COOCH_5$; 2,4 - 2,7 (m, 2H) $CH_2-C=H$; 2,95 (t, 2H) $-S-CH_2-$; 3,5 (d, 2H) $CH_2OH$; 3,5 - 3,6 (m, 2H) $N-CH_2$; 4,05 (q, 2H) $CO_2CH_2CH_3$
Rf-wert: Cyclohexan/aceton = 2:1 : 0,34

**Beispiel 4**

**2(1-Thia-5-äthoxycarbonyl-pentyl)-4-formyl-Δ1-pyrrolin VII (n = 2)**

Eine Lsg. von 1,63 9 DMSO (Dimethylsulfoxid) abs. [(20,9 mMol, 1,48 ml] in 25 ml $CH_2Cl_2$ abs. wird unter $N_2$-Strom auf - 60°C abgekühlt. 1,2 g Oxalylchlorid (9,6 mMol, 0,84 ml) werden zugetropft und noch 10 Min. gerührt. Danach wird eine Lsg. von 2,25 g 2(1-Thia-5-äthoxycarbonyl-pentyl)-4-hydroxymethyl-Δ1-pyrrolin VI in 15 ml $CH_2Cl_2$ abs. zugetropft und noch 20 Min bei -60°C gerührt. Bei dieser Temp. werden 4,4 g Triethylamin (43,5 mMol, 6,06 ml) zugetropft, und die Lsg. noch 30 Min. nachgerührt. Danach wird die Lsg. bei -10°C bis + 20°C durch Zugabe von ethanol. HCl neutralisiert. Nach dem Einengen wird der Rückstand in EE (Ethylether) aufgenommen, das ausgefallene Ammoniumsalz abfiltriert, mit EE gut gewaschen und Filtrat erneut eingeengt.
Ausbeute: 2,0 g hellgelbes Öl (90 % d. Th.) VII $C_{12}H_{19}NSO_3$ MG = 257
Rf-wert: Cyclohexan/Aceton 2:1: 0,38
MS: 257 (Molmasse)
NMR ($CDCl_3$): δ ppm 9,9 (d; 1H)

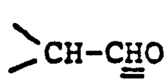

**Beispiel 5**

**2(1-Thia-5-äthoxycarbonyl-pentyl)-4-(3-oxo-1-octenyl) Δ1-pyrrolin IX (n = 2)**

0,35 g NaH 55%ig wird in 20 ml DME (1,2-Dimethoxyethan) abs. unter $M_2$-Strom suspendiert und 15 Min. bei R. T. gerührt. Dazu tropft man bei R. T. 1,64 g 2-Oxoheptyl-phosphonsäuredimethylester in 20 ml DME abs.. (weißes Na-Salz fällt aus.) Bei max. 30°C wird ca. 1 Std. nachgerührt. Anschl. werden 2,0 g 2(1-Thia-5-äthoxycarbonyl-pentyl)-4-formyl-Δ1-pyrrolin VII in 20 ml DME rasch zugegeben. Es wird ca. 2 Std. bei R. T. gerührt.

Nach beendeter Reaktion wird mit Eisessig angesäuert (pH 5) und eingeengt. Rückstand in EE aufgenommen und mit $H_2O$ und NaCl ges. gewaschen. EE-Phase getrocknet und eingeengt.
Ausbeute: 1,92 g (73,6 % d. Th.) hellgelbes Öl IX $C_{19}H_{31}O_3NS$ MG: 353
MS: 353 (Molmasse)
NMR ($CDCl_3$): δ ppm
1,2 (t, 3H) $CO_2CH_2\underline{CH_3}$; 1,5 - 1,9 (m, 2H) $CH_2$-$CH_2$; 2,1 - 2,9 (m, 7H) $\underline{CH_2}CO$, =$C\underline{H}$-, -$\underline{CH_2}$-C=, $\underline{CH_2}$-$CO_2CH_2CH_3$; 3,0 (t, 2H) -S-$CH_2$; 3,5 - 3,65 (m, 2H) N-$CH_2$; 4,05 (q, 2H) $CO_2\underline{CH_2}CH_3$;5,9 - 6,9 (ABX-system, 2H) -HC=CH
Rf-wert: Cyclohexan/Aceton = 2: 1: 0,57-

**Beispiel 6**

**2(1-Thia-5-äthoxycarbonyl-pentyl)-4(3-hydroxy-1 -octenyl)-Δ1-pyrrolin I (n = 2)**

Zu 1,0 g 2(Thia-5-äthoxycarbonyl-pentyl-4(3-oxo-1-octenyl)-Δ1-pyrrolin, IX Beispiel 5 gelöst in 15 ml Toluol abs. werden unter $N_2$-Strom 17 ml einer 0,45 molaren Bis-Isobornyloxy-isopropyloxy-aluminat-Lösung langsam zugetropft. Es wird bei R. T. gerührt. Nach 2 Std. aufgearbeitet. Reaktionsprodukt mit EE versetzt und 3 x mit gesättigter Natriumhydrogentartratlsg. extrahiert, Natriumhydrogentartrat-Phase nochmals mit EE gewaschen. Vereinigte EE-Phasen getrocknet und eingeengt.
Rohausbeute: 2,87 g hellgelbes Öl I
Über Merck-Fertigsäule Größe C /-Kieselgel (Korngr: 0,063 - 0,2 mm) mit Laufmittel Cyclohexan I/EE 1 chromatographiert:
Frakt. 25 - 44 = 450 mg (3'β-Epimeres)
Frakt. 54 - 125 = 440 mg (3'α-Epimeres)
Ausbeute: $C_{19}H_{33}NSO_2$- MG = 355 g 0,9 g (90 % d. Th.) helles Öl
MS: 355 (Molmasse)
NMR ($CDCl_3$): δ ppm Spektren für α-und β-Epimeres im Rahmen der üblichen Auflösung 60 MHz-$^1$H identisch. Signale wie Beispiel 5, zusätzliche Signale bei:
3,8 - 4,1 (m, 1H) $\underline{CH}$-OH, 5,45-5,6 (m, 3H) -CH=CH- (ABX-System bei 5.9 - 6.9 v. Beispiel 5 fehlt!)
Rf-Wert: Cyclohexan / Essigester = 1:1:
3'β-Epimeres 0,39
3'α-Epimeres 0,26

**Beispiel 7**

**2(1-Thia-4-äthoxycarbonyl-butyl)-4-hydroxy-methyl -Δ1-pyrrolin VI (n=1)**

Zu 31,5 g (0,23 Mol) $K_2CO_3$ in 100 ml DMF abs. werden 59,5 g 4-Hydroxymethyl-pyrrolidin-2-thion IV (Beispiel 2) gelöst in 200 ml DMF abs. zugetropft. Anschl. 30 Min. bei R. T. gerührt ($N_2$-Strom). Dann auf 100° C erhitzt und 87,7g (0,45 Mol) Brombuttersäureethylester gelöst in 65 ml DMF zugetropft. Nach 2 1/2 h bei 100°C Reaktion beendet. DC in Cyclohexan 2 / Aceton 1, Jod anfärben. Dann bei 80° C DMT abgezogen und Rückstand in EE und $H_2O$ aufgenommen und mit 2NHCl neutralisiert. $H_2O$-Phase abgetrennt und EE-Phase noch mehrmals mit $H_2O$ und NaClges. gewaschen. Vereinigte EE-Phasen getrocknet und eingeengt. Über Kieselgel Merck (0,063-0,2 mm) und Laufmittel Cyclohexan 3/Aceton-1 (später Cyclohexan 2/Aceton 1) chromatographiert.
Ausbeute: 71 g (64 % d. Th.) gelbes Öl $C_{11}H_{19}NSO_3$ MG: 245
NMR($CDCl_3$): δ ppm 1,2 (t, aH) $CO_2CH_2\underline{CH_3}$; 1,8 - 2,1 (m, 2H) -$CH_2$-; 2,2 - 2,8 (m,5H) $\underline{CH}$-$CH_2OH$, $\underline{CH_2}$-$COOC_2H_5$, = C-$CH_2$; 3,0 (t, 2H -S-$CH_2$-; 3,5 (d, 2H) $\underline{CH_2}OH$; 3,5 - 3,7 (m, 2H) N-$CH_2$; 4,05 (q, 2H) $COO\underline{CH_2}$;
$R_f$-wert: Cyclohexan / Aceton = 2:1: 0,31

**Beispiel 8**

**2(Thia-4-äthoxycarbonyl-butyl)-4-formyl-Δ 1-pyrrolin VII (n=1)**

Eine Lsg. von 3,0 g DMSO abs. frisch (39,2 m Mol 2,7 ml) in 35 ml $CH_2Cl_2$ abs. wird unter $N_2$-Strom auf -60°C abgekühlt. 2,25 g Oxalylchlorid (18 m Mol; 1,57 ml) werden zugetropft und noch 10 Min. gerührt. Danach wird eine Lsg. von 4 g 2(1-Thia-5-äthoxycarboxyl-butyl) -4-hydroxymethyl-Δ 1-pyrrolin VI (Beispiel 7) in 20 ml $CH_2Cl_2$ zugetropft und noch 20 Min. bei -60° C gerührt. Bei dieser Temp. werden 8,2 g Triethylamin (81,5 m Mol, 11,3 ml) zugetropft und die Lsg. noch 30 Min. nachgerührt. Danach wird die Lsg. bei 10°C bis + 20°G mit ethanol. HCl neutral gestellt. Nach dem Einengen wird in EE aufgenommen und das ausgefallene Ammonchlorid

abfiltriert, mit EE gut gewaschen und nochmals eingeengt.
Ausbeute: 3,5 g hellgelbes Öl (88 % d. Th.) VII $C_{11}H_{17}NSO_3$ MG: 243
$R_f$-wert: Cyclohexan / Aceton = 2: 1 0,34
MS: 243 (Molmasse)
NMR ($CDCl_3$): δ ppm 9,8 (d; 1H)

$>CH-\underline{\underline{CH}}O$

**Beispiel 9 a**

**2(1-Thia-4-äthoxycarbonyl-butyl)-4(3-oxo-1-octenyl) Δ 1-pyrrolin IX (n = 1)**

1,05 g NaH 55 %ig werden in 30 ml DME abs. suspendiert und 15 Min. gerührt. Dazu tropft man bei R. T. 4,92 g 2-Oxoheptylphosphonsäuredimethylester (22,2m Mol) gelöst in 50 ml DME abs. (Weißes Na-Salz fällt aus). Bei max. 30° C wird 1 Std. nachgerührt. Anschließend werden 5,4 g 2(1-Thia-4-äthoxycarbonyl-butyl)-4-formyl-Δ1-pyrrolin VII (Beispiel 8) gelöst in 30 ml DME abs. rasch zugegeben. Nach ca. 3 Std. Reaktion beendet, mit Essigsäure angesäuert (pH5). D.C. in Cyclohexan 2 / Aceton 1. Reaktionsprodukt eingeengt, in EE aufgenommen und 3 x mit NaCl ges. extrahiert. EE-Phase getrocknet und eingeengt. Über Kieselgel-Säule Merck (Korngr. 0,04-0,063 mm) mit Laufmittel Cyclohexan 5 / Aceton 1 chromatographiert.
Ausbeute: Fract. 53 - 73 = 5,2 g (70 % d. Th.) helles Öl IX $C_{18}H_{29}O_3NS$ MG 339
MS: 339 (Molmasse)
NMR ($CDCl_3$): δ ppm 1,2 (t, 3H) $COO\underline{CH_2}CH_3$; 1,8 - 2,9 (m, 7H) CH, $CH_2$; 3,05 (t, 2H) $-CH_2-S$; 3,5-3,65 (m, 2H) N-$CH_2$; 4,05 (q, 2H) $CO_2\underline{CH_2}\,CH_3$; 5,9 - 6,9 (ABX-system, 2H) -CH = CH
$R_f$-wert: Cyclohexan / Aceton = 2:1: 0,53

**Beispiel 9 b - 9 i**

In Analogie zu Beispiel 9 a lassen sich aus den entsprechenden Phosphonaten der allg. Formel VIII und 2(1-Thia-4-äthoxycarbonyl-butyl)-4-formyl-Δ1-pyrrolin VII (n = 1) die Verbindungen 9 b - 9 i der Formel IX darstellen.

| Beispiel Nr. 9 | $R^2$= | $R_f$-Werte (Cyclohexan 2/Aceton 1) |
|---|---|---|
| b) | 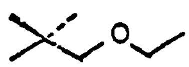 | 0,42 |
| c) | 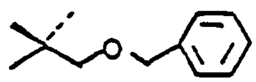 | 0,50 |
| d) | 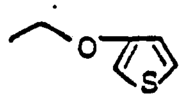 | 0,69 Cyclohexan 1 / Aceton 1) |
| e) | 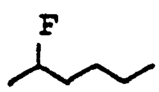 | 0,55 |
| f) | 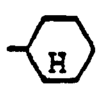 | 0,51 |
| g | 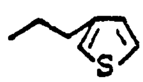 | 0,13 |

| Beispiel Nr. 9 | $R^2$= | $R_f$-werte (Cyclohexan 2/Aceton 1) |
|---|---|---|
| h) | 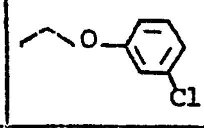 | 0,37 |
| i) | 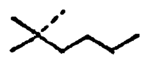 | 0,42 |

**Beispiel 10 a**

**2(1-Thia-4-äthoxycarbonyl-butyl)-4-(3-hydroxy-1-octenyl)-Δ1-pyrrolin I (n = 1)**

Zu 600 mg 2(1-Thia-4-äthoxycarbonyl-butyl-4(3-oxo-1-octenyl) Δ1-pyrrolin IX (Beispiel 9a) gelöst in 10 ml Toluol abs. werden unter $N_2$-Strom 10,8 ml einer 0,45 molaren Bis-isobornyloxy-isopropyloxy-aluminat-Lösung getropft. Die Relation ist nach 2 1/2 Std. beendet und wird aufgearbeitet. Dazu mit ca. 100 ml EE versetzt und 2 x mit gesättigter Natriumhydrogentartrat-Lösung extrahiert. Die Natriumhydrogentartrat-Phase noch 1 x mit EE gewaschen und vereinigte EE-Phasen getrocknet und eingeengt.

Über Merck-Fertigsäure-Größe C/Kieselgel (Korngr. 0,063-0,2 mm) mit Laufmittel Cyclohexan 1/EE1 chromatographiert.

Frakt. 186 - 290 = 190 mg (3'β-Epimeres)
Frakt. 350 - 490 = 220 mg (3'α-Epimeres)
<u>Ausbeute</u>: $C_{18}H_{31}NSO_3$ MG 341 0,41 g (68 % d. Th.) helles Öl
<u>MS</u>: 341 (Molmasse)
<u>NMR($CDCl_3$)</u>: δ ppm Spektren für α- und β-Epimeres im Rahmen der üblichen Auflösung 60 MHz-1H identisch. Signale wie <u>Beispiel 9 a</u>, zusätzliche Signale bei: 3,8 - 4,15 (m, 1H) CH-ON, 5,45 - 5,55 (m, 2H) -CH=CH- (ABX-system beim 5,9 - 6,9 v. Beispiel 9 a fehlt)
<u>Rf-wert</u>: Cyclohexan/Essigester = 1:1: 3'α Epimeres 0,38
3'β Epimeres 0,25

**Beispiel 10 b - 10 i**

In Analogie zu Beispiel 10 a lassen sich aus den Verbindungen der Beispiele 9 b - 9 i durch Reduktion mit Bis-isobornyloxy-isopropyloxy-aluminat die Verbindungen 10 b - 10 i herstellen (Formel I n = 1)

| Beispiel Nr. 10 | $R^2$= | NMR-Daten (ppm) charakteristische Signale | MS (Molmasse) | Rf-werte Essigester, Cyclohexan 1:1 ß/α-Epimer |
|---|---|---|---|---|
| b | 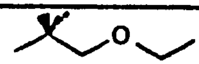 | 0,9 (s, 6H) C $(CH_3)_2$; 1,15 (t, 3H) $-OCH_2\underline{CH_3}$; 1,2 (t, 3H) $COOCH_3CH_2$; 3,3 (s, 2H) $-OCH_2$; 3,5 (q, 2H) $-OCH_2\underline{CH_3}$; 4,05 (q, 2H) $COOCH_2\underline{CH_3}$; 5,5 - 5,6 (m, 2H) olefin. Protonen | 371 | 0,39 / 0,29 |
| c | 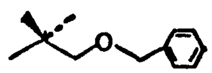 | 0,9 (d, 6H) $CH_3$; 1,2 (t, 3H) $OCH_2\underline{CH_3}$ 3,05 (t, 2H) S-$CH_2$; 3,25 (s, 2H) $-CH_2O-$; 3,5 - 3,65 (m, 2H) -N-$CH_2$- 4,45 (s, 2H) $\underline{CH_2}O$ — $C_6H_5$ 5,5 - 5,6 (m, 2H) CH=CH; 7,2 (s, 5H) aromat. Prot. | 433 | 0,51/0,41 |
| d | 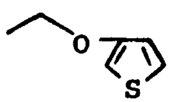 | 3,9 (d, 2H) $CH_2O$-thienyl 3,05 (t, 2H)-S-$CH_2$; | 383 | 0,25/0,19 |

| Beispiel Nr. 10 | $R^2$= | NMR-Daten (ppm) charakteristische Signale | MS (Molmasse) | Rf-werte Essigester, Cyclohexan 1:1 ß/α-Epimer |
|---|---|---|---|---|
| d Fortsetzung | | 5,5 - 5,65 (m, 2H) olefin. Protonen<br>6,1 - 7,3 (dreifaches m, 3H) Thiophen | | |
| e | 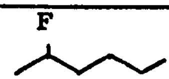 | 3,05 (t, 2H) $-S-CH_2$;<br>3,5 - 3,65 (m, 2H) $N-CH_2$;<br>5,5 - 5,6 (m, 2H) olefin. Protonen | 359 | 0,34/0,24 |
| f | 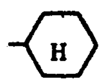 | 1,2 (t, 3H) $COOCH_2CH_3$;<br>1,1 - 2,0 (m, 3H) -CH,<br>$-CH_2-$ 3,05 (t, 2H) $-S-CH_2-$<br>3,55 - 5,6 (m, 2H) olefin. Protonen | 353 | 0,36/0,23 |
| g | 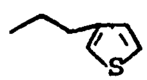 | 3,05 (t, 2H); 3,5 - 3,65 (m, 2H) $N-CH_2$; 5,5 - 5,6 | 381 | 0,25/0,16 |

| Beispiel Nr. 10 | $R^2$= | NMR-Daten (ppm) charakteristische Signale | MS (Molmasse) | Rf-werte Essigester, Cyclohexan 1:1 ß/α-Epimer |
|---|---|---|---|---|
| g Fortsetzung | | (m, 2H) olefin. Protonen; 6,8 - 7,3 (3H) Thiophen | | |
| h | 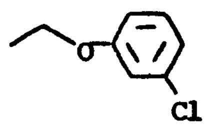 | 3,85 (d, 2H) $CH_2$-O-Ph 3,05 (t, 2H) S-$CH_2$- 5,5 - 5,6 (m, 2H) olefin. Protonen; 6,0 - 7,3 (m, 4H) aromat. Protonen | 411 | 0,33/0,18 |
| i | 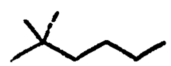 | 0,85 (s, 6H) $CH_3$; 3,05 (t, 2H) S-$CH_2$; 3,5 - 3,65 (m, 2H) olef. Protonen | 369 | 0,42/0,28 |

**Beispiel 11 a**

**Natriumsalz des 2(1-Thia-4-carboxy-butyl)-4(3-hydroxy -1-octenyl)-Δ1-pyrrolins I (n = 1, $R^1$ = Na⊕)**

136 mg (0,4 m Mol) 2(1-Thia-4-äthoxycarbonyl-butyl)-4(3-hydroxy-1-octenyl)Δ 1-pyrrolin (Beispiel 10 a) werden in 30 ml 80 % Äthanol gelöst. Zu dieser Lösung gibt man unter Rühren eine Lösung von 67 mg Natrium in 4 ml Äthanol. Man rührt 3 Std. bei 35° C unter Argon., filtriert die Lösung über Aktivkohle und entfernt das Lösungsmittel im Vakuum bei 10° C (Gefriertrocknung). Man erhält das Natriumsalz des Δ1-Pyrro-linderivates I als farbloses Pulver.

IR-Bande: KBr-Verreibung -COO ⊖ 1600 $cm^{-1}$

**Beispiel 11 b**

**Kaliumsalz des 2(1-Thia-5-carboxy-pentyl)-4(3-hydroxy1-octenyl)-Δ1-pyrrolins I (n = 2, $R^1$ = K⊕)**

134 mg reines 2(1-Thia-5-äthoxycarbonyl-pentyl)-4 (3-hydroxy-1-octenyl)-Δ1-pyrrolin (Beispiel 6), 1,1 ml 0,5 M Kaliumhydroxidlösung und 2 ml Methanol läßt man unter Inertgas 24 Stunden bei Raumtemperatur stehen. Das Methanol wird im Vakuum abgezogen und die wässrige Lösung des Kaliumsalzes gefriergetrocknet. Man erhält das Kaliumsalz des Δ1-Pyrrolinderivates I als farbloses Pulver.

IR-Bande: KBr-Verreibung -COO⊖ 1595 $cm^{-1}$

**Beispiel 11 c**

**Triäthylammoniumsalz des 2(1-Thia-4-carboxy-butyl)-4 (3-hydroxy-1-octenyl)-Δ1-pyrrolins I (n = 1, $R^1$ = HN⊕$(C_2H_5)_3$)**

Eine wässrige Lösung von 50 mg des Natriumsalzes von Beispiel 11 a wird auf eine Säule mit 15 g Amberlite CG - 50 (Triäthylammonium-Form) gegeben. Man eluiert mit einer 3 %igen wässrigen Lösung von Triäthylammoniumcarbonat. Durch Gefriertrocknen des Eluats erhält man das Produkt als kristallines Pulver (Zers. 70° C). In Analogie zu den Beispielen 11a bis 11 c lassen sich aus den Verbindungen der Beispiele 10 b bis 10 i durch alkalische Esterverseifung und ggf. Chromatographie an Ionenaustauschern die entsprechenden Alkali- oder Ammoniumsalze herstellen.

**Beispiel 12**

**2(1-Thia-4-isopropyloxycarbonyl-butyl)-4(3-hydroxy-4,4 dimethyl-1-octenyl)- Δ1-pyrrolin I (n = 1, $R^1$ = $CH(CH_3)_2$)**

369,5 mg (1 mMol) 2(1-Thia-4-äthoxycarbonyl-butyl)-4-(3-hydroxy-4,4-dimethyl -1-octenyl)-Δ 1-pyrrolin (Beispiel 10 i) werden in 40 ml Isopropanol abs. gelöst und mit 50 mg gepulvertem und gut getrocknetem Kaliumcarbonat versetzt. Es wird 1 Std. gerührt. Das Lösungsmittel wird in Vakuum abgezogen, der Rückstand in Essigester aufgenommen, EE mit Wasser gewaschen, getrocknet, i. Vak. Lsgm. entfernt.

Ausbeute: 345 mg helles Öl

3'β-Epimeres Rf-wert Cyclohexan/EE 1:1 0,49

3'α-Epimeres Rf-wert " " " 0,34

NMR ($CDCl_3$): δ ppm 4,95 (Septett,1H) C<u>H</u>$(CH_3)_2$; 1,2 (d,6H) $CH(\underline{CH_3})_2$

**Patentansprüche** für die Vertragsstaaten BE CH DE FR GB IT LI LU NL SE

1) Verbindungen der Formel I

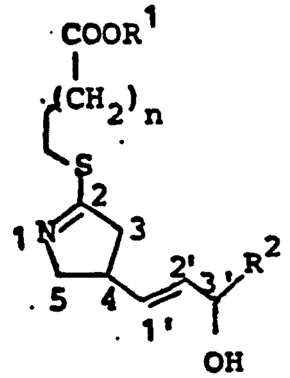

I

in welcher bedeuten:

$R^1$ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen oder ein physiologisch verträgliches Metall-, $NH_4$- oder, ein Ammoniumion, das sich von einem primären, sekundären oder tertiären Amin ableitet, oder ein Tetraalkylammoniumion,

$R^2$ einen Phenylrest, der 1-3-fach substituiert sein kann mit Halogen, Trifluormethyl, Alkyl und/ oder Alkoxy mit je 1-6 C-Atomen, einen unsubstituierten cycloaliphatischen Kohlenwasserstoffrest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen, oder einem Phenylmethyloxyrest,

b) Halogen, unsubstituiertem Cycloalkyl mit 3-7 C-Atomen, Phenyl oder einem α- oder β-Thienyl- oder α- oder β-Furylrest, die ihrerseits 1-3-fach substituiert sein können mit Halogen, Trifluormethyl, Alkyl und/oder Alkoxy mit je 1-6 C-Atomen,

c) einem unsubstituierten Cycloalkoxyrest mit 3-7 Kohlenstoffatomen, einem Phenoxy- oder einem α- oder β-Thienyloxyrest, wobei die genannten aromatischen Reste ihrerseits 1 -3-fach substituiert sein können mit Halogen, Trifluormethyl, Alkyl und/oder Alkyloxy mit je 1-6 C-Atomen, und

n die Zahl 0, 1, 2, 3 oder 4.

2. Verfahren zur Herstellung von Verbindungen der Formel gemäß Anspruch 1, dadurch gekennzeichnet, daß man

a) eine Verbindung der Formel VII

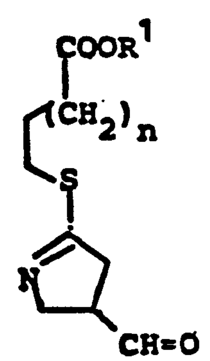

VII

worin $R^1$ und n die zur Formel I in Anspruch 1 genannten Bedeutungen haben, mit einem Phosphonat der Formel VIII

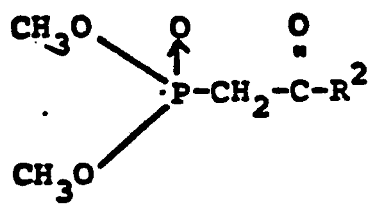

VIII

worin $R^2$ die zur Formel I in Anspruch 1 genannten Bedeutungen hat, umsetzt zu einem Enon der Formel IX

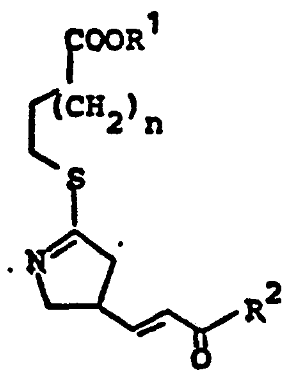

IX

b) das erhaltene Enon zu einem Epimerengemisch der Alkohole der Formel I reduziert,

c) gegebenenfalls das erhaltene Epimerengemisch der Alkohole der Formel I in das α - und β-Isomere auftrennt,

d) gegebenenfalls eine Verbindung der Formel I, worin $R^1$ nicht Wasserstoff oder ein Kation bedeutet, verseift zu einer Verbindung der Formel I, worin $R^1$ Wasserstoff oder ein physiologisch verträgliches Kation wie in Anspruch 1 definiert, bedeutet,

e) gegebenenfalls in einer Verbindung der Formel I, worin $R^1$ Wasserstoff oder ein physiologisch verträgliches Metall-, $NH_4$-oder Ammoniumion bedeutet, das sich von einem primären, sekundären oder tertiären Amin ableitet, und $R^2$ und n die zur Formel 1 gegebenen Bedeutungen haben, das Kation $R^1$ gegen ein anderes under die Definition von $R^1$ in Anspruch 1 fallendes Kation austauscht.

3. Verfahren zur Herstellung von Verbindungen der Formel VII

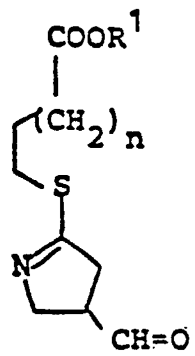

VII

worin $R^1$ und n die zur Formel I in Anspruch 1 genannten Bedeutungen haben, dadurch gekennzeichnet, daß man

a) das Lactam der Formel II

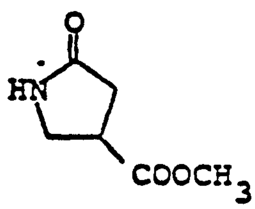

II

nach üblichen Methoden umwandelt in das Thiolactam der Formel III,

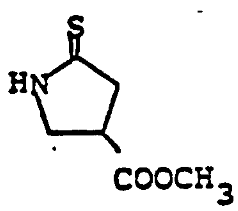

III

b) das Thiolactam der Formel III durch Reduktion der Esterfunktion in den Thiolactamalkohol der Formel IV überführt,

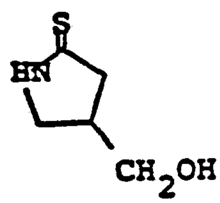

IV

c) den Thiolactamalkohol der Formel IV mit einer Verbindung der Formel V,
Hal - $CH_2$ - $CH_2$ - $(CH_2)_n$-$COOR^1$
worin $R_1$ und n die zur Formel I genannte Bedeutung haben und Hal Jod, Chlor oder Brom bedeutet, alkyliert zu einer Verbindung der Formel VI

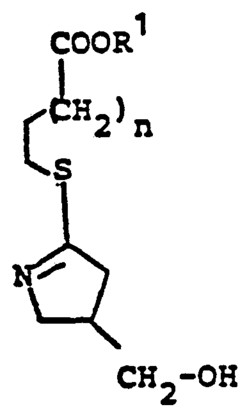

VI

d) und die erhaltene Verbindung der Formel VI oxydiert zu einem Aldehyd der Formel VII,

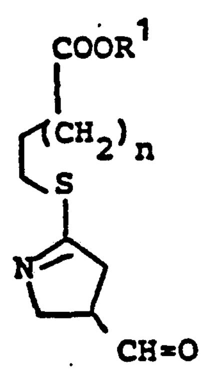

VII

worin $R^1$ und n die oben genannte Bedeutung haben.
4. Verbindungen der Formel VI

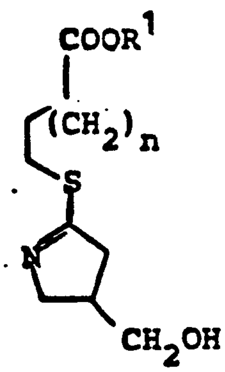

. VI

worin $R^1$ und n die zur Formel I in Anspruch 1 genannten Bedeutungen haben.

5. Verbindungen der Formel VII

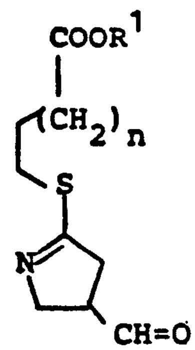

VII

worin $R^1$ und n die zur Formel I in Anspruch 1 genannten Bedeutungen haben.

6. Verbindungen der Formel IX

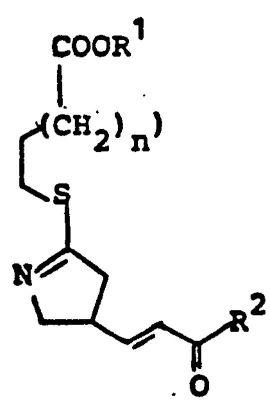

IX

worin $R^1$, R und n die zur Formel I in Anspruch 1 genannten Bedeutungen haben.

7. Verfahren zur Herstellung von Arzneimitteln, dadurch gekennzeichnet, daß man eine Verbindung der Formel I, gegebenenfalls mit üblichen pharmazeutischen Trägern und/ oder Stabilisatoren, in eine therapeutisch geeignete Darreichungsform bringt.

8. Arzneimittel, gekennzeichnet durch den Gehalt an einer Verbindung der Formel I in Mischung mit einem pharmazeutisch üblichen Träger und/oder Stabilisator.

9. Verbindungen der Formel I zur Verwendung als Arzneimittel.

**Patentansprüche** für den Vertragsstaat AT

1.Verfahren zur Herstellung von Verbindungen der Formel I

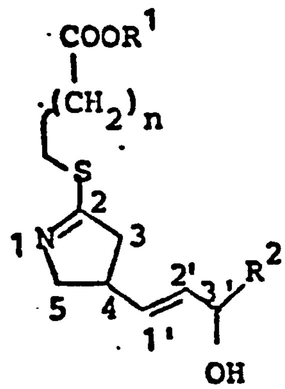

I

in welcher bedeuten:

$R^1$ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen oder ein physiologisch verträgliches Metall-, $NH_4$- oder ein Ammoniumion, das sich von einem primären, sekundären oder tertiären Amin ableitet, oder ein Tetraalkylammoniumion,

$R^2$ einen phenylrest, der 1-3-fach substituiert sein kann mit Halogen, Trifluormethyl, Alkyl und/oder Alkoxy mit je 1 - 6 C-Atomen, einen unsubstituierten cycloaliphatischen Kohlenwasserstoffrest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen, oder einem Phenylmethyloxyrest,

b) Halogen, unsubstituiertem Cycloalkyl mit 3-7 C-Atomen, Phenyl oder einem α- oder β-Thienyl- oder α- oder β-Furylrest, die ihrerseits 1-3-fach substituiert sein können mit Halogen, Trifluormethyl, Alkyl und/oder Alkoxy mit je 1-6 C-Atomen,

c) einem unsubstituierten Cycloalkoxyrest mit 3-7 Kohlenstoffatomen, einem Phenoxy- oder einem α- oder β-Thienyloxyrest, wobei die genannten aromatischen Reste ihrerseits 1-3-fach substituiert sein können mit Halogen, Trifluormethyl, Alkyl und/oder Alkyloxy mit je 1-6 C-Atomen, und

n die Zahl 0, 1, 2, 3 oder 4,

dadurch gekennzeichnet, daß man

a) eine Verbindung der Formel VII

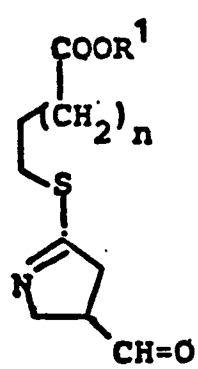

VII

worin $R^1$ und n die zur Formel I in Anspruch 1 genannte Bedeutung haben, mit einem Phosphonat der Formel VIII

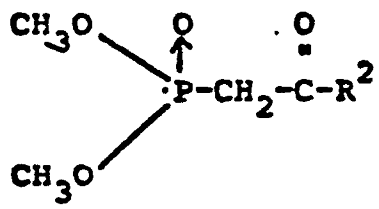

VIII

worin $R^2$ die zur Formel I in Anspruch 1 genannten Bedeutungen hat, umsetzt zu einem Enon der Formel IX

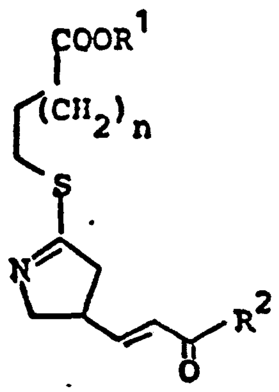

IX

b) das erhaltene Enon zu einem Epimerengemisch der Alkohole der Formel I reduziert,

c) gegebenenfalls das erhaltene Epimerengemisch der Alkohole der Formel I nach üblichen Methoden in das α - und β-Isomere auftrennt,

d) gegebenenfalls eine Verbindung der Formel I, worin $R^1$ nicht Wasserstoff oder ein Kation bedeutet, verseift zu einer Verbindung der Formel I, worin $R^1$ Wasserstoff oder ein physiologisch verträgliches Kation wie oben zur Formel I definiert bedeutet,

e) gegebenenfalls in einer Verbindung der Formel I, worin $R^1$ Wasserstoff oder ein physiologisch verträgliches Metall-, $NH_4$-oder Ammoniumion bedeutet, das sich von einem primären, sekundären oder tertiären Amin ableitet, und $R^2$ und n die zur Formel 1 gegebenen Bedeutungen haben, das Kation $R^1$ gegen ein anderes unter die oben angegebene Definition von $R^1$ fallendes Kation austauscht.

2. Verfahren zur Herstellung von Verbindungen der Formel VII

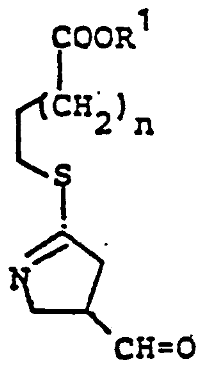

VII

worin $R^1$ und n die zur Formel I in Anspruch 1 genannte Bedeutung haben dadurch gekennzeichnet, daß man
a) das Lactam der Formel II

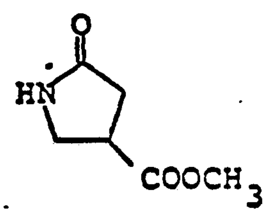

II

umwandelt in das Thiolactam der Formel III

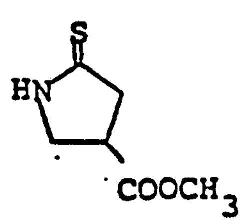

III

b) das Thiolactam der Formel III durch Reduktion der Esterfunktion in den Thiolactamalkohol der Formel IV überführt,

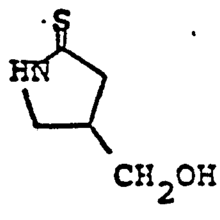

IV

c) den Thiolactamalkohol der Formel IV mit einer Verbindung der Formel V,
Hal - $CH_2$ - $CH_2$ - $(CH_2)_n$-COOR[1]
worin $R_1$ und n die zur Formel I genannte Bedeutung haben und Hal Jod, Chlor oder Brom bedeutet, alkyliert zu einer Verbindung der Formel VI

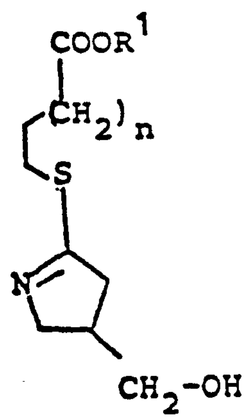

VI

d) und die erhaltene Verbindung der Formel VI oxydiert zum Aldehyd der Formel VII,

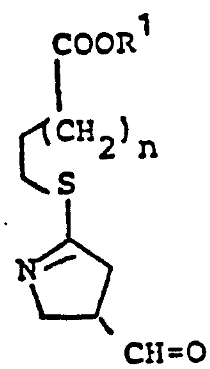

VII

worin $R^1$ und n die oben genannte Bedeutung haben.
3. Verfahren zur Herstellung von Verbindungen der Formel IX

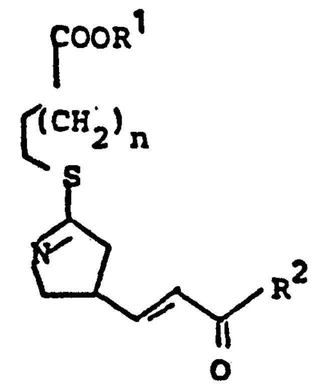

IX

worin $R^1$, $R^2$ und n die zur Formel I in Anspruch 1 genannten Bedeutungen haben, dadurch gekennzeichnet, daß man eine Verbindung der Formel VII

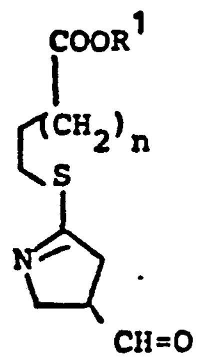

VII

worin $R^1$ die zur Formel I in Anspruch 1 genannte Bedeutung hat, umsetzt mit einem Phosphonat der Formel VIII

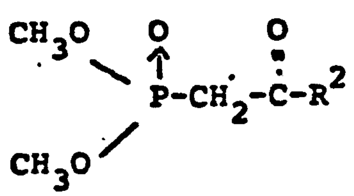

VIII

worin $R^2$ die zur Formel I gegebenen Bedeutungen hat.

**Claims** for the Contracting States: BE, CH, DE, FR, GB, IT, LI, LU, NL,

SE.

1. A compound of the formula I

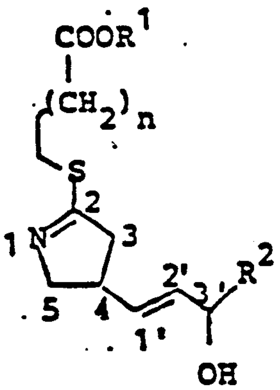

I

in which $R^1$ denotes hydrogen, a straight-chain or branched alkyl radical with up to 8 carbon atoms, a straight-chain or branched unsaturated aliphatic hydrocarbon radical with 3 to 6 carbon atoms, a cycloaliphatic hydrocarbon radical with 3 to 7 carbon atoms, an araliphatic hydrocarbon radical with 7 to 9 carbon atoms or a physiologically acceptable metal ion, $NH_4$ ion or ammonium ion which is derived from a primary, secondary or tertiary amine, or a tetraalkylammonium, $R^2$ denotes a phenyl radical, which can be mono-, di- or tri-substituted in the nucleus by halogen, trifluoromethyl, alkyl and/or alkoxy with in each case 1-6 C-atoms, or denotes an unsubstituted cycloaliphatic hydrocarbon radical with 3-8 carbon atoms, a straight-chain or branched alkyl radical with up to 8 carbon atoms or a straight-chain or branched unsaturated aliphatic hydrocarbon radical with 3 to 8 carbon atoms, it being possible for the aliphatic radicals in turn to be substituted by

a) a straight-chain or branched alkoxy radical with up to 6 carbon atoms or a straight-chain or branched alkenyloxy or alkynyloxy radical with 3 to 6 carbon or a phenylmethoxy radical, b) halogen, unsubstituted cycloalkyl with 3-7 C atoms, phenyl or an α- or β-thienyl or α- or β-furyl radical, which can in turn be mono-, di- or tri-substituted by halogen, trifluoromethyl, alkyl and/or alkoxy with in each case 1-6 C atoms, or c) an unsubstituted cycloalkoxy radical with 3-7 carbon atoms, a phenoxy or α- or β-thienyloxy radical it being possible for the aromatic radicals mentioned to be in turn mono-, di- or tri-substituted by halogen, trifluoromethyl, alkyl and/or alkoxy with in each case 1-6 C atoms, and n denotes the number 0, 1, 2, 3 or 4.

2. A process for the preparation of a compound of the formula I as claimed in claim 1, which comprises

a) reacting a compound of the formula VII

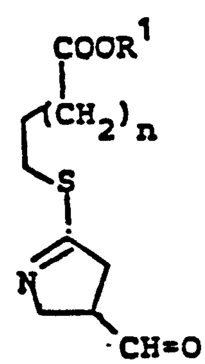

VII

in which $R^1$ and n have the meanings given in the case of formula I in claim 1, with a phosphonate of the formula VIII

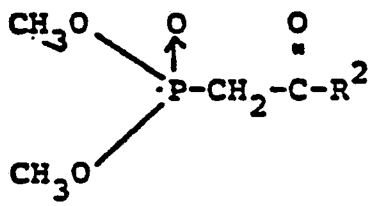

VIII

in which $R^2$ has the meanings given in the case of formula I in claim 1, to give an enone of the formula IX

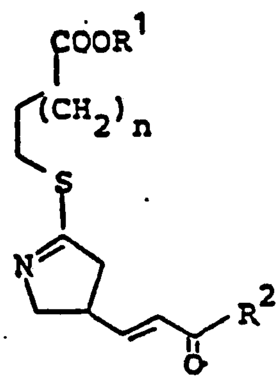

IX

b) reducing the resulting enone to give an epimer mixture of the alcohols of the formula I,

c) where appropriate, separating the resulting epimer mixture of the alcohols of the formula I into the α- and β-isomers

d) where appropriate, hydrolyzing a compound of the formula I in which $R^1$ does not denote hydrogen or a cation to give a compound of the formula I in which $R^1$ denotes hydrogen or a physiologically acceptable as defined in claim 1,

e) where appropriate, replacing the cation $R^1$ in a compound of the formula I in which $R^1$ denotes hydrogen or a physiologically acceptable metal ion, $NH_4$ ion or ammonium ion. which is derived from a primary, secondary or tertiary amine and $R^2$ and n have the meanings given in the case of formula I, by another cation falling within the definition of $R^1$ in claim 1.

3. A process for the preparation of a compound of the formula VII

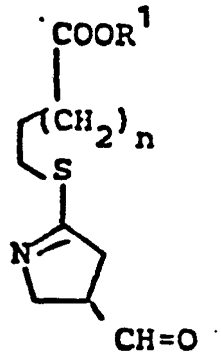

VII

in which $R^1$ and n have the meaning given in formula I in claim 1, which comprises
a) converting the lactam of the formula II

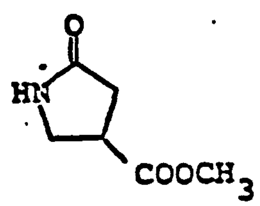

II

into the thiolactam of the formula III

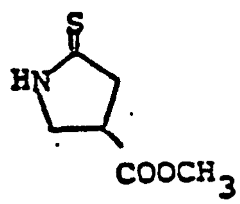

III

by conventional methods,
b) converting the thiolactam of the formula III into the thiolactam alcohol of the formula IV

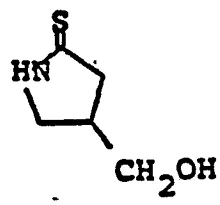

IV

by reduction of the ester group,

c) alkylating the thiolactam alcohol of the formula IV with a compound of the formula V

$Hal-CH_2-CH_2-(CH_2)_n-CCOR^1$

in which $R^1$ and n have the meaning given in the case of formula I and Hal denotes iodine, chlorine or bromine, to give a compound of the formula VI

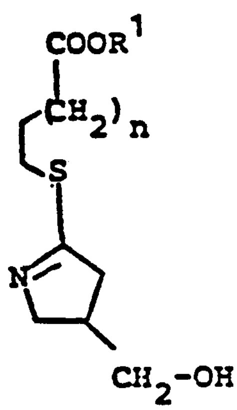

VI

d) oxidizing the resulting compound of the formula VI to an aldehyde of the formula VII

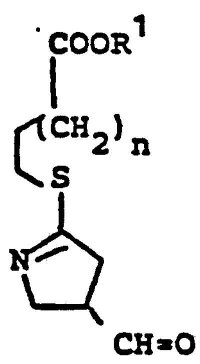

VII

in which $R^1$ and n have the meaning given above.

4. A compound of the formula VI

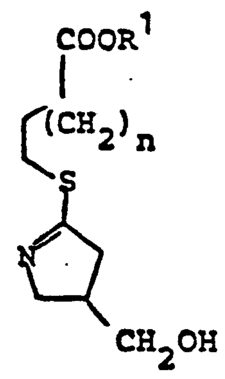

VI

in which $R^1$ and n have the meanings given in the case of formula I in claim 1.

5. A compound of the formula VII

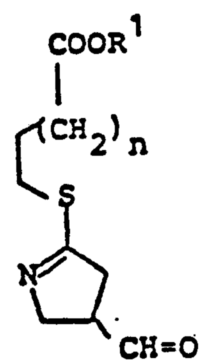

VII

in which $R^1$ and n have the meanings given in the case of formula I in claim 1.

6. A compound of the formula IX

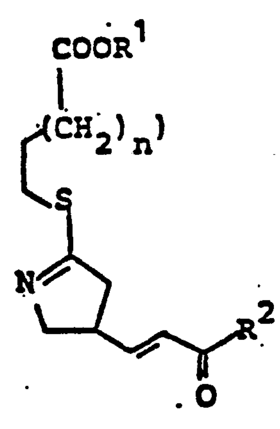

IX

in which $R^1$, $R^2$ and n have the meanings given in the case of formula I in claim 1.

7. A process for the preparation of medicaments, which comprises converting a compound of the formula I into a therapeutically suitable form of administration, if appropriate together with conventional pharmaceutical excipients and/or stabilizers.

8. A medicament which contains a compound of the formula I mixed with a pharmaceutically conventional excipient and/or stabilizer.

9. A compound of the formula I for use as a medicament.

**Claims** for the Contracting State: Austria:

1. A process for the preparation of a compound of the formula I

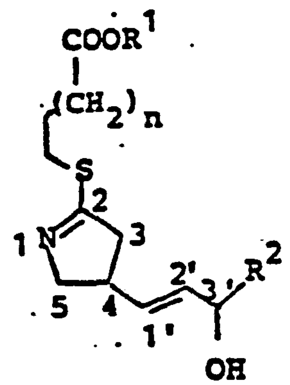

I

in which $R^1$ denotes hydrogen, a straight-chain or branched alkyl radical with up to 8 carbon atoms, a straight-chain or branched unsaturated aliphatic hydrocarbon radical with 3 to 6 carbon atoms, a cycloaliphatic hydrocarbon radical with 3 to 7 carbon atoms, an araliphatic hydrocarbon radical with 7 to 9 carbon atoms or a physiologically acceptable metal ion, $NH_4$ ion or ammonium ion which is derived from a primary, secondary or tertiary amine, or a tetraalkylammonium, $R^2$ denotes a phenyl radical, which can be mono-, di- or tri-substituted in the nucleus by halogen, trifluoromethyl, alkyl and/or alkoxy with in each case 1-6 C-atoms, or denotes an unsubstituted cycloaliphatic hydrocarbon radical with 3-8 carbon atoms, a straight-chain or branched alkyl, radical with up to 8 carbon atoms or a straight-chain or branched unsaturated aliphatic hydrocarbon radical with 3 to 8 carbon atoms, it being possible for the aliphatic radicals in turn to be substituted by

a) a straight-chain or branched alkoxy radical with up to 6 carbon atoms or a straight-chain or branched alkenyloxy or alkynyloxy radical with 3 to 6 carbon or a phenylmethoxy radical, b) halogen, unsubstituted cycloalkyl with 3-7 C atoms, phenyl or an α- or β-thienyl or α- or β-furyl radical, which can in turn be mono-, di- or tri-substituted by halogen, trifluoromethyl, alkyl and/or alkoxy with in each case 1-6 C atoms, or c) an unsubstituted cycloalkoxy radical with 3-7 carbon atoms, a phenoxy or α - or β-thienyloxy radical it being possible for the aromatic radicals mentioned to be in turn mono-, di- or tri-substituted by halogen, trifluoromethyl, alkyl and/or alkoxy with in each case 1-6 C atoms, and n denotes the number 0, 1, 2, 3 or 4, which comprises

a) reacting a compound of the formula VII

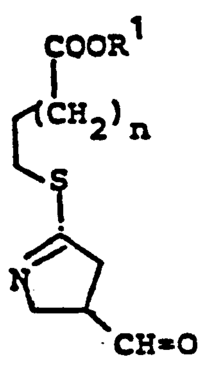

VII

in which $R^1$ and n have the meaning given in the case of formula I in claim 1, with a phosphonate of the formula VIII

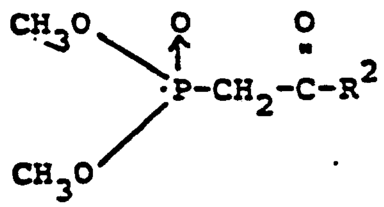

VIII

in which $R^2$ has the meanings given in the case of formula I in claim 1, to give an enone of the formula IX

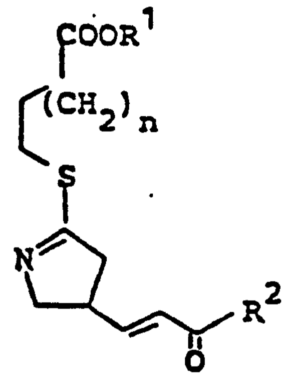

IX

b) reducing the resulting enone to give an epimer mixture of the alcohols of the formula I,

c) where appropriate, separating the resulting epimer mixture of the alcohols of the formula I into the α- and β-isomers by conventional methods,

d) where appropriate, hydrolyzing a compound of the formula I in which $R^1$ does not denote hydrogen or a cation to give a compound of the formula I in which R denotes hydrogen or a physiologically acceptable cation as defined above in formula I,

e) where appropriate, replacing the cation $R^1$ in a compound of the formula I in which $R^1$ denotes hydrogen or a physiologically acceptable metal ion, $NH_4$ ion or ammonium ion wwhich is derived from a primary, secondary or tertiary amine and $R^2$ and n have the meanings given in the case of formula I, by another cation falling within the above definition of $R^1$.

2. A process for the preparation of a compound of the formula VII

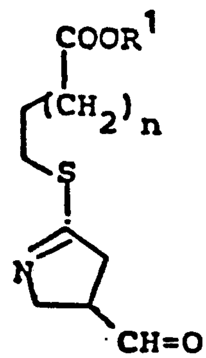

VII

in which $R^1$ and n have the meaning given in the case of formula I in claim 1, which comprises a) converting the lactam of the formula II

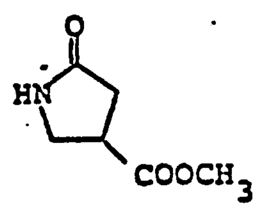

II

into the thiolactam of the formula III

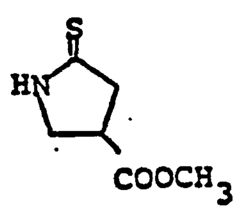

III

b) converting the thiolactam of the formula III into the thiolactam alcohol of the formula IV

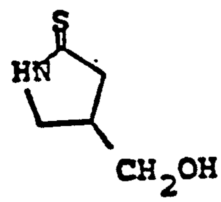

IV

by reduction of the ester group,

c) alkylating the thiolactam alcohol of the formula IV with a compound of the formula V

$Hal-CH_2-CH_2-(CH_2)$ -$COOR^1$

in which $R^1$ and n have the meaning given in the case of formula I and Hal denotes iodine, chlorine or bromine, to give a compound of the formula VI

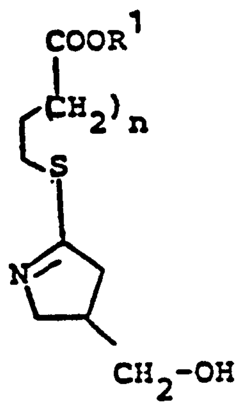

VI

and

d) oxidizing the resulting compound of the formula VI to an aldehyde of the formula VII

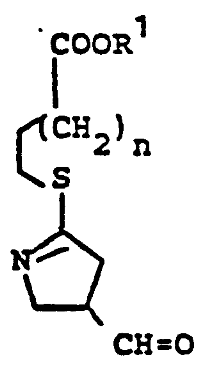

VII

in which $R^1$ and n have the meaning given above.

3. A process for the preparation of a compound of the formula IX

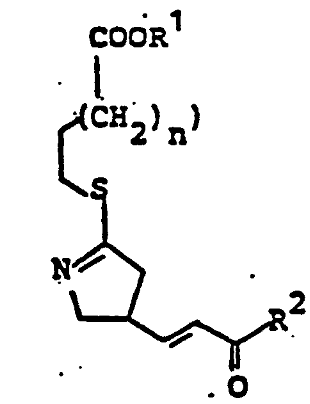

IX

in which $R^1$, $R^2$ and n have the meanings given in the case of formula I in claim, which comprises reacting a compound of the formula VII

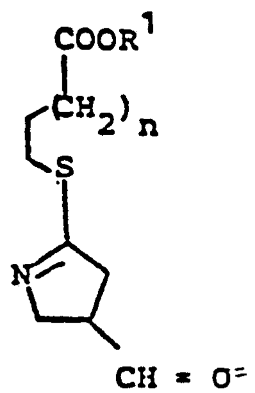

VII

in which $R^1$ and n have the meanings given in the case of formula I in claim 1 with a phosphonate of the formula VIII

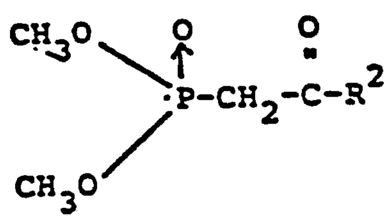

VIII

in which $R^2$ has the meanings given in the case of formula I in claim 1.

**Revendications** pour les Etats contractants: BE, CH, DE, FR, GB, IT, LI,

LU, NL, SE.

1. Composés de formule I:

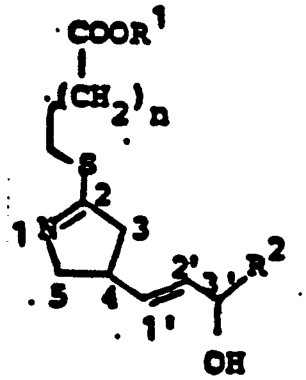

I

dans laquelle:

$R^1$ représente l'hydrogène, un groupe alkyle à chaîne droite ou ramifiée ayant jusqu'à 8 atomes de carbone, un groupe hydrocarboné aliphatique, insaturé, à chaîne droite ou ramifiée, ayant de 3 à 6 atomes de carbone, un groupe hydrocarboné cycloaliphatique ayant de 3 à 7 atomes de carbone, un groupe hydrocarboné araliphatique ayant de 7 à 9 atomes de carbone ou un ion métallique physiologiquement acceptable, un ion $NH_4$ ou un ion ammonium qui dérive d'une amine primaire, secondaire ou tertiaire, ou un ion tétraalkylammonium,

$R^2$ représente un groupe phényle qui peut être de 1 à 3 fois substitué par un halogène, un groupe trifluorométhyle, alkyle et/ou alcoxy ayant chacun de 1 à 6 atomes de carbone, un groupe hydrocarboné cycloaliphatique nonsubstitué ayant de 3 à 8 atomes de carbone, un groupe alkyle à chaîne droite ou ramifiée ayant jusqu'à 8 atomes de carbone, un groupe hydrocarboné aliphatique insaturé, à chaîne droite ou ramifiée, ayant de 3 à 8 atomes de carbone, les groupes aliphatiques pouvant, de leur côté, être substitués par:

a) un groupe alcoxy à chaîne droite ou ramifiée ayant jusqu'à 6 atomes de carbone ou un groupe alcényloxy ou alcynyloxy à chaîne droite ou ramifiée ayant de 3 à 6 atomes de carbone, ou un groupe phénylméthyloxy,

b) un halogène, un groupe cycloalcoxy non-substitué ayant de 3 à 7 atomes de carbone, un groupe phényle ou un groupe α- ou β-thiényle ou α- ou β-furyle, qui, de leur côté, peuvent être de 1 à 3 fois substitués par un halogène, un groupe trifluorométhyle, alkyle et/ou alcoxy ayant chacun de 1 à 6 atomes de carbone,

c) un groupe cycloalkyle non-substitué ayant de 3 à 7 atomes de carbone, un groupe phénoxy ou un groupe α- ou β-thiényloxy, auquel cas les groupes aromatiques nommés peuvent, de leur côté, être de 1 à 3 fois substitués par un halogène, un groupe trifluorométhyle, alkyle et/ou alcoxy ayant chacun de 1 à 6 atomes de carbone, et

n est le nombre 0, 1, 2, 3 ou 4.

2. Procédé pour la préparation de composés de formule I selon la revendication 1, caractérisé en ce que:

a) on fait réagir un composé de formule VII

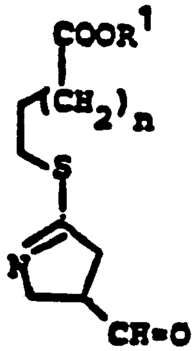

VII

dans laquelle $R^1$ et n ont les significations indiquées pour la formule I dans la revendication 1, avec un phosphonate de formule VIII:

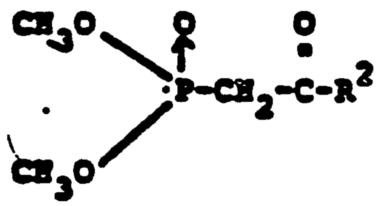

VIII

dans laquelle $R^2$ a les significations indiquées pour la formule I dans la revendication 1, pour obtenir une énone de formule IX:

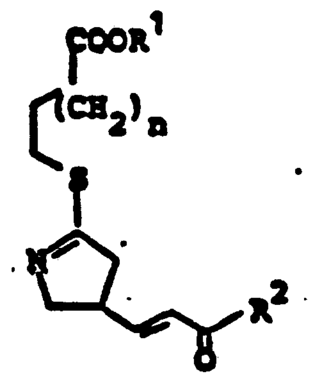

IX

b) on réduit l'énone obtenue en un mélange d'épimères des alcools de formule I,

c) on sépare éventuellement le mélange d'épimères des alcools de formule I obtenu en les isomères α et β,

d) on saponifie éventuellement un composé de formule I, dans lequel $R^1$ ne représente ni l'hydrogène ni un cation, en un composé de formule I, dans lequel $R^1$ represente l'hydrogène ou un cation physiologiquement acceptable tel que défini dans la revendication 1,

e) dans un composé de formule I, dans lequel $R^1$ représente l'hydrogène ou un ion métallique physiologiquement acceptable, un ion $NH_4$ ou un ion ammonium qui dérive d'une amine primaire, secondaire ou tertiaire, et $R^2$ et n ont les significations indiquées pour la formule I on échange éventuellement le cation $R^1$ contre un autre cation répondant à la définition de $R^1$ dans la revendication 1.

3. Procédé pour la préparation de composés de formule VII:

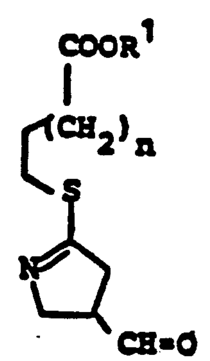

VII

dans laquelle R[1] et n ont les significations indiquées pour la formule I dans la revendication 1, caracterise en ce que

a) on transforme le lactame de formule II:

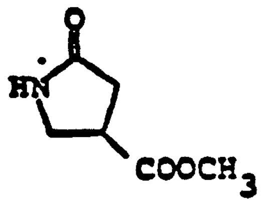

II

selon des procédés usuels, en le thiolactame de formule III:

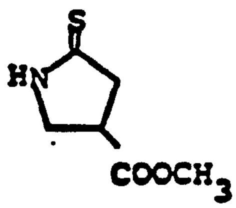

III

b) on convertit le thiolactame de formule III en le thiolactame alcool de formule IV par réduction de la fonction ester,

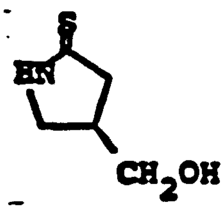

IV

c) on alkyle le thiolactame alcool de formule IV avec un composé de formule V:

$Hal - CH_2 - CH_2 - (CH_2)_n-COOR^1$

dans laquelle R[1] et n ont les significations indiquées pour la formule I et Hal représente l'iode, le chlore ou le brome, en un composé de formule VI:

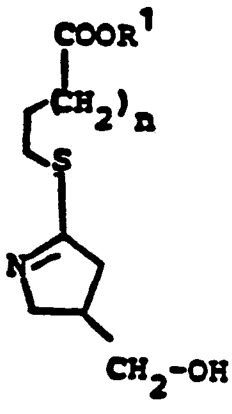

VI

d) et on oxyde le composé de formule VI obtenu en un aldéhyde de formule VII:

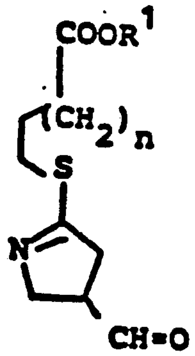

VII

dans alquelle $R^1$ et n ont les significations indiquées ci-dessus.

4. Composés de formule VI:

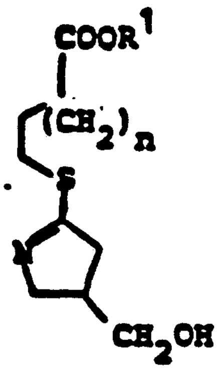

VI

dans laquelle $R^1$ et n ont les significations indiquées pour la formule I dans la revendication 1.

5. Composés de formule VII:

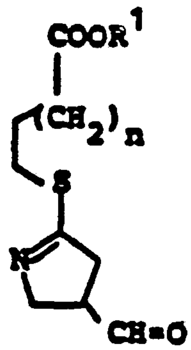

VII

dans laquelle $R^1$ et n ont les significations indiquées pour la formule I dans la revendication 1.

6. Composés de formule IX:

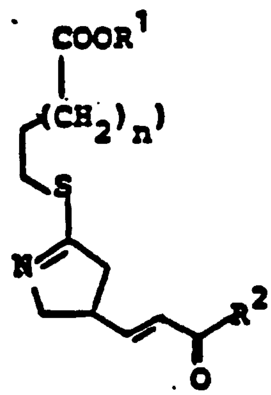

IX

dans laquelle $R^1$, $R^2$ et n ont les significations indiquées pour la formule I dans la revendication 1.

7. Procédé pour la préparation de médicaments, caractérisé en ce qu'on met un composé de formule I, sous une forme d'administration thérapeutiquement appropri-e, éventuellement avec des véhicules, et/ou des stabilisants pharmaceutiquement usuels.

8. Médicament, caractérisé par une teneur en un composé de formule I, mélangé avec un véhicule et/ou un stabilisant pharmaceutiquement usuel.

9. Composés de formule I utilisables comme médicaments.

**Revendications** pour l'Etat contractant: AT

1. Procédé pour la préparation de composés de formule I:

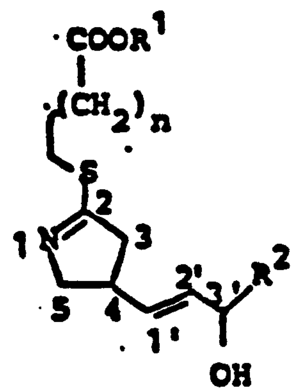

I

dans laquelle:

$R^1$ représente l'hydrogène, un groupe alkyle à chaîne droite ou ramifiée ayant jusqu'à 8 atomes de carbone, un groupe hydrocarboné aliphatique insaturé, à chaîne droite ou ramifiée, ayant de 3 à 6 atomes de carbone, un groupe hydrocarboné cycloaliphatique ayant de 3 à 7 atomes de carbone, un groupe hydrocarboné araliphatique ayant de 7 à 9 atomes de carbone ou un ion métallique physiologiquement acceptable, un ion $NH_4$ ou un ion ammonium qui dérive d'une amine primaire, secondaire ou tertiaire, ou un ion tétra-alkylammonium,

$R^2$ représente un groupe phényle qui peut être de 1 à 3 fois substitué par un halogène, un groupe trifluorométhyle, alkyle et/ou alcoxy ayant chacun de 1 à 6 atomes de carbone, un groupe hydrocarboné cycloaliphatique nonsubstitué ayant de 3 à 8 atomes de carbone, un groupe alkyle à chaîne droite ou ramifiée ayant jusqu'à 8 atomes de carbone, un groupe hydrocarboné aliphatique insaturé, à chaîne droite ou saturée, ayant de 3 à 6 atomes de carbone, les groupes aliphatiques pouvant, de leur côté, être substitués par:

a) un groupe alcoxy à chaîne droite ou ramifiée ayant jusqu'à 6 atomes de carboné ou un groupe alcényloxy ou alcynyloxy à chaîne droite ou ramifiée ayant de 3 à 6 atomes de carbone, ou un groupe phénylméthyloxy,

b) un halogène, un groupe cycloalkyle non-substitué ayant de 3 à 7 atomes de carbone, un groupe phényle ou un groupe α- ou β-thiényle ou α- ou β-furyle qui, de leur côté, peuvent être de 1 à 3 fois substitués par un halogène, un groupe trifluorométhyle, alkyle et/ou alcoxy ayant chacun de 1 à 6 atomes de carbone,

c) un groupe cycloalcoxy non-substitué ayant de 3 à 7 atomes de carbone, un groupe phénoxy ou un groupe α- ou β-thiényloxy, auquel cas les groupes aromatiques nommés peuvent, de leur côté, être de 1 à 3 fois substitués par un halogène, un groupe trifluorométhyle, alkyle et/ou alcoxy ayant chacun de 1 à 6 atomes de carbone, et

n est le nombre 0, 1, 2, 3 ou 4,

lequel procédé est caractérisé en ce que:

a) on fait réagir un composé de formule VII:

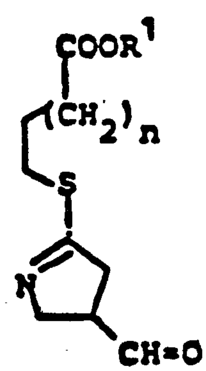

VII

dans laquelle $R^1$ et n ont les significations indiquées pour la formule I dans la revendication 1, avec un phosphonate de formule VIII:

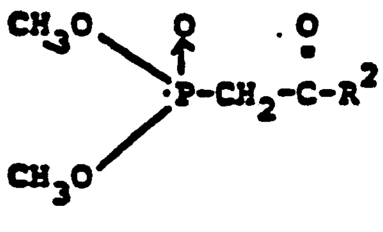

VIII

dans laquelle $R^2$ a les significations indiquées pour la formule I dans la revendication 1, pour obtenir une énone de formule IX:

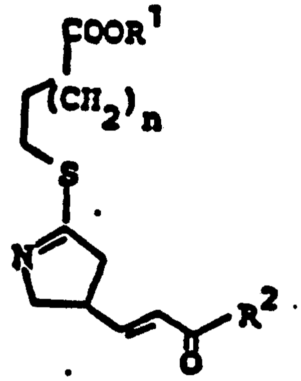

IX

b) on réduit l'énone obtenue en un mélange d'épimères des alcools de formule I,

c) éventuellement, on sépare le mélange d'épimères des alcools de formule I, obtenu, en les isomères $\alpha$ et $\beta$, selon des procédés usuels,

d) on saponifie éventuellement un composé de formule I, dans lequel $R^1$ n'est ni l'hydrogène, ni un cation, en un composé de formule I, dans lequel $R^1$ représente l'hydrogène ou un cation physiologiquement acceptable, tel que défini pour la formule I,

e) dans un composé de formule I, dans lequel $R^1$ représente l'hydrogène ou un ion métallique physiologiquement acceptable, un ion $NH_4$ ou un ion ammonium qui dérive d'une amine primaire, secondaire ou tertiaire, et $R^2$ et n ont les significations indiquées pour la formule I, on échange éventuellement le cation $R^1$ contre un autre cation repondant à la définition de $R^1$ donnée ci-dessus.

2. Procédé pour la préparation de composés de formule VII:

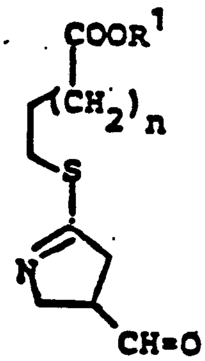

VII

dans laquelle R[1] et n ont les significations indiquées pour la formule I dans la revendication 1, caracterisé en ce que:

a) on transforme le lactame de formule II:

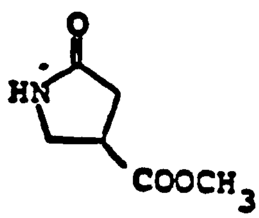

II

en le thiolactame de formule III:

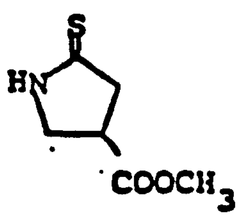

III

b) on convertit le thiolactame de formule III en le thiolactame alcool de formule IV, par réduction de la fonction ester,

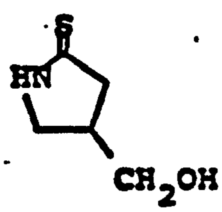

c) on alkyle le thiolactame alcool de formule IV avec un composé de formule V:
Hal - $CH_2$ - $CH_2$ - $(CH_2)_n$-$COOR^1$
dans laquelle $R^1$ et n ont les significations indiquées pour la formule I et Hal représente l'iode, le chlore ou le brome, pour obtenir un composé de formule VI:

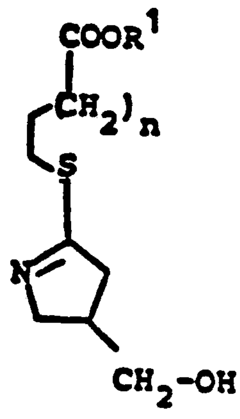

VI

d) et on oxyde le composé de formule VI obtenu en un aldéhyde de formule VII:

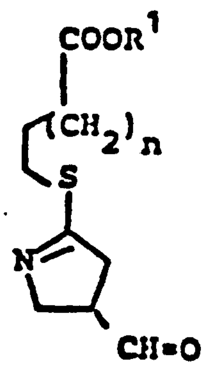

VII

dans laquelle $R^1$ et n ont les significations indiquées ci-dessus.
3. Procédé pour la préparation de composés de formule IX:

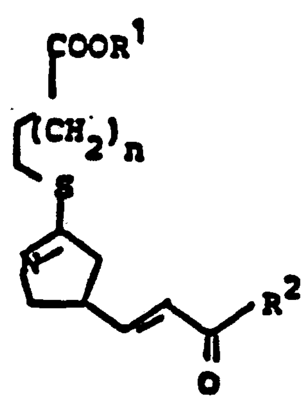

IX

dans laquelle $R^1$, $R^2$ et n ont les significations indiquées pour la formule I dans la revendication 1 caractérisé en ce qu'on fait réagir un composé de formule VII:

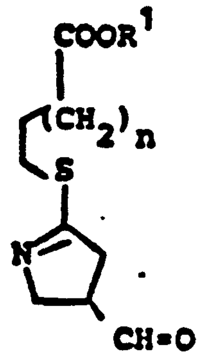

VII

dans laquelle $R^1$ a les significations indiquées pour la formule I dans la revendication 1, avec un phosphonate de formule VIII:

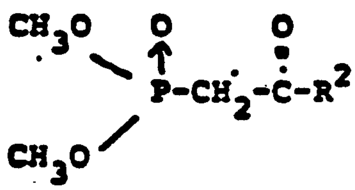

VIII

dans laquelle $R^2$ a les significations indiquées pour la formule I.